# EUROPEAN PATENT APPLICATION

(11) **EP 3 885 816 A1**
(43) Date of publication of application: **29.09.2021**
(21) Application number: 20181686.5
(22) Date of filing: 23.06.2020
(51) Int. Cl.: G02B 21/36, G06K 9/20, G06T 3/40, G01N 21/47, A61B 5/00

(54) **MICROSCOPIC IMAGING STITCHING APPARATUS AND METHOD THEREOF**

(30) Priority: 23.03.2020 CN 202010206582
(71) Applicant: CAL-COMP BIG DATA, INC, Shenkeng, New Taipei City 222 (TW)
(72) Inventor: HUANG, Yuan-Peng, 222 NEW TAIPEI CITY (TW)
(74) Representative: 2K Patentanwälte Blasberg Kewitz & Reichel

(57) **Abstract**

A microscopic imaging stitching apparatus used for detecting a skin to be tested (1) includes an optical cavity (10), a beam splitter (20), an image sensor (30), a displacement sensor (40), and a control circuit (50). The optical cavity (10) contacts the skin to be tested (1) and receives a reflected light (100) reflected from the skin to be tested (1). The beam splitter (20) receives the reflected light (100) through a microscope lens (121), and outputs a light to be tested to the image sensor (30) and the displacement sensor (40) along two different directions. The control circuit (50) receives an image signal (103) of the image sensor (30) and a displacement signal (104) of the displacement sensor (40), and stitches a plurality of the image signals (103) into a stitched image (106) according to a plurality of the displacement signals (104). The present disclosure further provides a microscopic imaging stitching method for detecting the skin to be tested (1).

## Description

### BACKGROUND

### Technical Field

The present disclosure relates to an imaging stitching apparatus and a method thereof, and more particularly to a microscopic imaging stitching apparatus and a method thereof that are applied to microscopic imaging and can exhibit a curvature of a skin to be tested.

### Description of Related Art

The statements in this section merely provide background information related to the present disclosure and do not necessarily constitute prior art.

Common digital microscopic apparatus on market include handheld consumer electron microscopes, external microscope lens for smart phone, or desktop biological microscopes. They can only observe microscopic images with limited field of view (FOV) on surface of an object to be tested. An observe range is limited by an optical path design of an optical microscopy system or a size limitation of an electronic components (such as CCD or CMOS). If a magnification ratio of the lens selected by an observer is higher, the FOV will be smaller. At this time, if the area to be tested is larger than the FOV, the observer must move the digital microscope or move the object to be tested in different areas. For microscopic observation, the magnification ration of microscope is relatively higher than naked eye of normal human, a higher-level commercial biological microscope may allow to take several consecutive photos and display enlarged photos separately to observe and analyze details of each enlarged photo in a larger FOV. It is especially important for judging biological characteristics of a particular area. For example, analysis of a wide range of pathological features, or spread state of symptoms, etc.

Although the prior art can display the enlarged photos separately, there is no way to present a single microscopic image with several times the FOV of a digital microscopic apparatus for the largest organ "skin" in the human body. Further, each of the aforementioned prior art photos is a two-dimensional planar photo. If the surface to be observed is non-planar, such as human skin, whether it is facial skin, limb skin, or body skin, it is a three-dimensional curved surface instead of a flat surface. If only display flat images, it may distort geometric features on the skin due to perspective distortion, such as barrel distortion or pincushion distortion, which may easily cause misjudgment during use. Therefore, the prior art digital microscopic apparatus cannot meet the needs of the aforementioned use scenarios.

Therefore, how to design a microscopic imaging stitching apparatus and method thereof, in particular to solve technical problems in prior art that cannot overcome the perspective distortion of skin's geometric features and cannot present a single microscopic image with several times FOV of single digital microscopic apparatus.

### SUMMARY

One of the purposes of the present disclosure is to provide a microscopic imaging stitching apparatus that solves the technical problems in prior art, so it can present a single microscopic image with several times FOV of single digital microscopic apparatus and can overcome the perspective distortion of skin's geometric features. Thereby achieving the purpose of facilitate the analysis of large-scale microscopic images and improving an accuracy of analysis.

In order to achieve the one of the purposes, the microscopic imaging stitching apparatus used for detecting a skin to be tested includes an optical cavity, a beam splitter, an image sensor, a displacement sensor, and a control circuit. The optical cavity has a frustum of a cone with a first plane and a second plane arranged parallel to each other, an area of the first plane is smaller than an area of the second plane, and the area of the first plane contacts the skin to be tested, the second plane emits light to the first plane and receives a reflected light reflected from the skin to be tested. The beam splitter receives the reflected light through a microscope lens, and outputs a first light to be tested and a second light to be tested along two different directions. The image sensor receives the first light to be tested, and outputs an image signal. The displacement sensor receives the second light to be tested, and outputs a displacement signal. The control circuit is coupled to the image sensor and the displacement sensor, the control circuit receives the image signal and the displacement signal, and stitches a plurality of the image signals into a stitched image according to a plurality of the displacement signals.

Further, the control circuit further receives at least one skin mark, each of the at least one skin mark includes a preset curvature corresponding to each skin to be tested, and the control circuit stitches the plurality of the image signals into the stitched image with the preset curvature according to the plurality of the displacement signals and the at least one skin mark.

Further, the microscopic imaging stitching apparatus further includes a triple-axis gyroscope is coupled to the control circuit, the triple-axis gyroscope is fixed in a housing and outputs a three-dimensional signal to the control circuit, the control circuit receives the image signal, the displacement signal, and the three-dimensional signal, and the control circuit stitches the plurality of the image signals into the stitched image with an actual curvature according to the plurality of the displacement signals and a plurality of the three-dimensional signals.

Further, the microscopic imaging stitching apparatus further includes a plurality of light-emitting units arranged on the second plane, the plurality of the light-emitting units emit light toward the first plane, a minimum angle between an extension line of one side of each of the plurality of the light-emitting units and the normal line of the first plane being 30°.

Further, when the control circuit receives one of the pluralities of the image signals, the control circuit determines whether another one of the pluralities of the image signals is continuously received according to a threshold value and at least one of the pluralities of the displacement signals.

Further, the threshold value is 2mm, and the field of view (FOV) of the image sensor is 3mm x 3mm.

Further, a ratio of a light intensity of the first light to be tested to a light intensity of the reflected light is 99%, 95%, or 90%, a ratio of a light intensity of the second light to be tested to a light intensity of the reflected light is 1%, 5%, or 10%.

The other one of the purposes of the present disclosure is to provide a microscopic imaging stitching method that solves the technical problems in prior art, so it can present a single microscopic image with several times FOV of single digital microscopic apparatus and can overcome the perspective distortion of skin's geometric features. Thereby achieving the purpose of facilitate the analysis of large-scale microscopic images and improving an accuracy of analysis.

In order to achieve the other one of the purposes, the microscopic imaging stitching method for detecting a skin to be tested includes following steps of: A frustum of a cone emits light to a skin to be tested. A beam splitter receives a reflected light reflected from the skin to be tested through a microscope lens, and outputs a first light to be tested and a second light to be tested along two different directions. An image sensor receives the first light to be tested, and outputs an image signal. A displacement sensor receives the second light to be tested, and outputs a displacement signal. A control circuit receives the image signal and the displacement signal, and stitches a plurality of the image signals into a stitched image according to a plurality of the displacement signals.

Further, the control circuit further receives at least one skin mark, each of the at least one skin mark includes a preset curvature corresponding to each skin to be tested, and the control circuit stitches the plurality of the image signals into the stitched image with the preset curvature according to the plurality of the displacement signals and the at least one skin mark.

Further, the control circuit further receives a three-dimensional signal, and the control circuit stitches the plurality of the image signals into the stitched image with an actual curvature according to the plurality of the displacement signals and a plurality of the three-dimensional signals.

Further, the microscopic imaging stitching apparatus further includes following step of: when the control circuit receives one of the plurality of the image signals, the control circuit determining whether another one of the plurality of the image signals is continuously received according to a threshold value and at least one of the plurality of the displacement signals.

Further, the threshold value is 2mm, and the field of view (FOV) of the image sensor is 3mm x 3mm.

Further, an area of 1mm inward around the FOV of the image sensor is an overlap area that one of the pluralities of the image signals and another image signal are stitched together.

Further, a ratio of a light intensity of the first light to be tested to a light intensity of the reflected light is 99%, 95%, or 90%, a ratio of a light intensity of the second light to be tested to a light intensity of the reflected light is 1%, 5%, or 10%.

Further, the microscopic imaging stitching apparatus further includes following step of: the frustum of a cone moves on the skin to be tested at a speed of Imm/s.

When using the microscopic imaging stitching apparatus of the present disclosure, the optical cavity receives the reflected light reflected from the skin to be tested through the microscope lens. And then the beam splitter outputs the first light to be tested to an image sensor (such as a CCD or CMOS), and outputs the second light to be tested to the displacement sensor (such as an optical XY two-dimensional sensor). Because of most of energy of the reflected light is distributed to the image sensor, it will not affect an establishment of subsequent stitching images. And because of the displacement sensor and the image sensor can be synchronously performed (if the control circuit determines that the displacement signal is larger than the threshold value), the control circuit can evenly and smoothly perform stitching processing on the plurality of image signals. In detail, the skin mark corresponding to each part of the skin to be tested can be inputted or preset to store before measurement. Because each skin mark includes the curvature of each part of the skin to be tested (for example, a curvature of chin skin of human is larger than a curvature of forehead skin of human). The control circuit can stitch the plurality of image signals into the stitched image with a curvature according to the plurality of displacement signals and the at least one skin mark. The curvature can be a preset value that conforms to most human skin types, and can enable the control circuit to obtain the stitched image with less perspective distortion than a traditional fully flat image. It effectively reduces the misjudgment of skin geometric features. If the triple-axis gyroscope coupled to the control circuit, the stitched image with precise curvature can be obtained even further.

For this reason, the microscopic imaging stitching apparatus that solves the technical problems in prior art, so it can present a single microscopic image with several times FOV of single digital microscopic apparatus and can overcome the perspective distortion of skin's geometric features. Thereby achieving the purpose of facilitate the analysis of large-scale microscopic images and improving an accuracy of analysis.

In order to further understand the techniques, means, and effects of the present disclosure for achieving the intended purpose. Please refer to the following detailed description and drawings of the present disclosure. The drawings are provided for reference and description only, and are not intended to limit the present disclosure.

### BRIEF DESCRIPTION OF DRAWINGS

FIG. 1 is a system architecture diagram of a microscopic imaging stitching apparatus of the present disclosure.
FIG. 2 is a schematic diagram of a first embodiment of the microscopic imaging stitching apparatus of the present disclosure.
FIG. 3 is a schematic diagram showing skin marks of the microscopic imaging stitching apparatus of the present disclosure.
FIG. 4 and FIG. 5 are schematic diagrams of the stitching images of the microscopic imaging stitching device of the present disclosure.
FIG. 6 is a schematic diagram of a second embodiment of the microscopic imaging stitching apparatus of the present disclosure.
FIG. 7 is a schematic diagram of facial skin imaging of the microscopic imaging stitching apparatus of the present disclosure.

### DETAILED DESCRIPTION

The embodiments of the present disclosure are described by way of specific examples, and those skilled in the art can readily appreciate the other advantages and functions of the present disclosure. The present disclosure may be embodied or applied in various other specific embodiments, and various modifications and changes can be made without departing from the spirit and scope of the present disclosure.

It should be understood that the structures, the proportions, the sizes, the number of components, and the like in the drawings are only used to cope with the contents disclosed in the specification for understanding and reading by those skilled in the art, and it is not intended to limit the conditions that can be implemented in the present disclosure, and thus is not technically significant. Any modification of the structure, the change of the proportional relationship, or the adjustment of the size, should be within the scope of the technical contents disclosed by the present disclosure without affecting the effects and the achievable effects of the present disclosure.

The technical content and detailed description of the present disclosure will be described below in conjunction with the drawings.

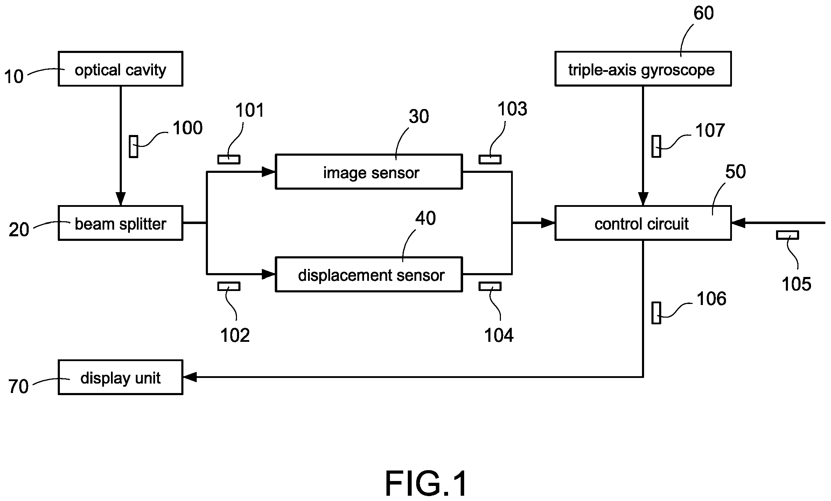

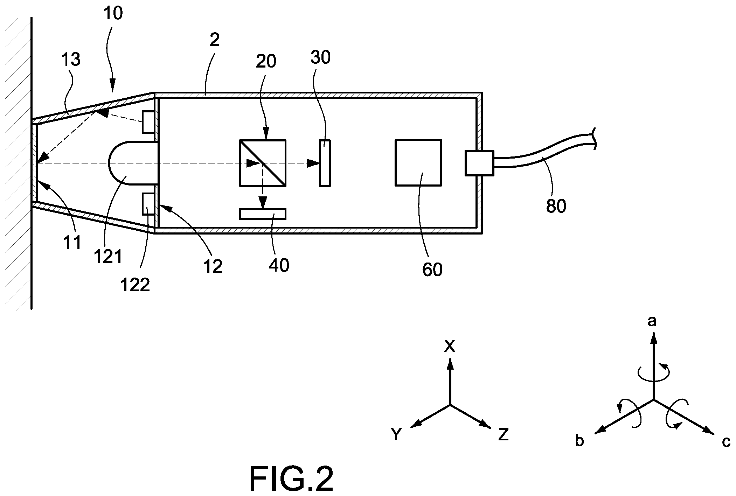

Please refer to FIG. 1 and FIG. 2. FIG. 1 is a system architecture diagram of a microscopic imaging stitching apparatus of the present disclosure. FIG. 2 is a schematic diagram of a first embodiment of the microscopic imaging stitching apparatus of the present disclosure.

In the first embodiment of the present disclosure, the microscopic imaging stitching apparatus used for detecting a skin to be tested 1 includes an optical cavity 10, a beam splitter 20, an image sensor 30, a displacement sensor 40, a control circuit 50, and a triple-axis gyroscope 60. The optical cavity 10 has a frustum of a cone with a first plane 11 and a second plane 12 arranged parallel to each other, an area of the first plane 11 is smaller than an area of the second plane 12, and the area of the first plane 11 contacts the skin to be tested 1, the second plane 12 emits light to the first plane 11 and receives a reflected light 100 reflected from the skin to be tested 1. Further, the microscopic imaging stitching apparatus of the present disclosure further includes a plurality of light-emitting units 122 arranged on the second plane 12, there has a microscope lens 121 penetrates the second plane 12, and the microscope lens 121 receives the reflected light 100. The plurality of the light-emitting units 122 emit light toward the first plane 11, and irradiate the skin to be tested 1 in contact with the first plane 11. A minimum angle between an extension line of one side of each of the plurality of the light-emitting units 122 and the normal line of the first plane 11 being 30°. An annular surface 13 that is opaque and is formed between one side of the first plane 11 and one side of the second plane 12, and the annular surface 13 is covered with an aluminum metal film for reflecting light. In the first embodiment of the present disclosure, the plurality of the light-emitting units 122 include a plurality of white light-emitting units (not shown) and a plurality of excitation light-emitting units (not shown) spaced at intervals. The plurality of the white light-emitting units does not emit light simultaneously with the plurality of the excitation light-emitting units, and an excitation light emitted by the plurality of the excitation light-emitting units between 350 nm and 400 nm, which is used to cause human skin to emit fluorescent light.

In particular, the specific wavelength optimized is 365 nm (the tolerance is plus and minus 5 nm), and the specific wavelength belongs to ultraviolet light (UV). As an example of propionibacterium acnes, which is prone to cause skin diseases, it is a group of bacteria commonly found in the pores of the skin surface and is one of the main causes of acne. The cleanliness of the pores on the skin also affects the chance of causing skin diseases, but the average pore size of the pores is only about 0.9 mm, which needs to be observed with a microscope. Generally, a common digital microscopic device using visible light as a main light source is difficult to effectively distinguish and observe the growth state of propionibacterium acnes in the pores and whether the propionibacterium acnes is effectively cleaned due to its wavelength limitation. For this purpose, a fluorescence microscope is required for the detection of propionibacterium acnes. The propionibacterium acnes produces a chemical called "porphyrin" in the metabolic process, and the chemical structure of the porphyrin may be excited by the ultraviolet light (the main absorption peak is 365 nm) to excite an orange fluorescence that belongs to visible light range (the main emission peak is 660 nm). Therefore, it is possible to indirectly know the growth of propionibacterium acnes in each skin region by observing the light intensity of the orange fluorescence, the size of the dot region of the orange fluorescence, and the dot distribution. The foregoing is merely illustrative, and there is not limited an application of the present disclosure.

The beam splitter 20 receives the reflected light 100 through the microscope lens 121, and outputs a first light to be tested 101 and a second light to be tested 102 along two different directions. Further, the beam splitter 20 outputs the first light to be tested 101 to the image sensor 30, and the beam splitter 20 outputs the second light to be tested 102 to the displacement sensor 40. In the first embodiment of the present disclosure, the beam splitter 20 may include a harmonic separator or a dichroic beam splitter. The beam splitter 20 reflects a part of the energy of the reflected light 100 belonging to a certain wavelength range to form the second light to be tested 102, and transmits another part of the energy of the reflected light 100 belonging to the certain wavelength range to form the first light to be tested 101. In the first embodiment of the present disclosure, a ratio of a light intensity of the first light to be tested 101 to a light intensity of the reflected light 100 is 99%, 95%, or 90%, a ratio of a light intensity of the second light to be tested 102 to a light intensity of the reflected light 100 is 1%, 5%, or 10%. That is, the relationship between the first light to be tested 101 and the second light to be tested 102 is 99%:1%, 95%:5%, or 90%:10%. Because most of energy of the reflected light 100 is distributed to the image sensor 30, it will not affect an establishment of subsequent stitching images. The beam splitter 20 can be used in fields such as fluorescence resonance energy transfer (FRET), real-time live-cell imaging, or fluorescence imaging. However, the foregoing is merely illustrative, and there is not limited an application of the present disclosure.

The image sensor receives the first light to be tested 101, and outputs an image signal 103. The image sensor 30 may be a photosensitive element composed of a CCD or a CMOS, and may include a micro lens structure to enhance the low-light sensing sensitivity of the CCD or the CMOS. In the first embodiment of the present disclosure, the field of view (FOV) formed by the microscope lens 121 and the image sensor 30 is 3mm x 3mm. In use, the first plane 11 of the frustum of a cone can be moved in a scanning manner against the surface of the object to be tested (such as skin on face). For example, the moving speed is 1mm/s, and the photos are taken continuously or discontinuously at the same time to obtain a photo set for stitching.

The displacement sensor 40 receives the second light to be tested 102, and outputs a displacement signal 104. The displacement sensor 40 may be an optical XY two-dimensional sensor. The optical XY two-dimensional sensor may include a low-resolution CMOS and a microcontroller (MCU). The fast processing characteristics of the low-resolution CMOS can be used to quickly determine the difference between two consecutive images before and after. The speed can reach 1500pc/s. This allows the MCU to know the direction of the image movement in the current FOV or the direction of the displacement sensor 40 through digital image correlation and tracking (DIC and tracking) related technologies, and then output a two-dimensional displacement signal 104 includes (X,Y) for subsequent applications, and each of the X and Y values represents a moving distance in each direction relative to a preset absolute zero. Because the first plane 11 contacts with the skin to be tested 1, another dimension Z is a fixed value and does not need to be detected (as shown in FIG. 2). It is worth mentioning that in sensing displacement with the XY two-dimensional, the main reason for not being able to choose other sensing methods, such as a physical triple-axis accelerometer, is that the displacement speed of the triple-axis accelerometer is very slow. Furthermore, the accuracy of accelerometers with low g specifications is also insufficient. Relatively, the accuracy of the optical XY two-dimensional sensor that can be used for optical mouse is sufficient for the application of microscopic imaging of skin. It can be achieved by directly splitting the reflected light 100 by using original light path, and it is easy to integrate the original design.

The control circuit 50 is coupled to the image sensor 30 and the displacement sensor 40, the control circuit 50 receives the image signal 103, the displacement signal 104, and at least one skin mark 105, and stitches a plurality of the image signals 103 into a stitched image 106 with a preset curvature according to the plurality of the displacement signals 104 and the at least one skin mark 105. The control circuit 50 outputs the stitched image 106 to a display unit 70. The preset curvature may be pre-build and pre-stored, and each skin mark 105 includes the preset curvature corresponding to each part of the skin to be tested 1, that is, the present disclosure may pre-build a virtual facial image. The control circuit 50 may be a circuit board including an image signal processor (ISP). In the first embodiment of the present disclosure, an area of 1mm inward around the FOV (for example, 3mm x 3mm) of the image sensor 30 is an overlap area that one of the pluralities of the image signals 103 and another image signal 103 are stitched together by microscopic imaging stitching. It can be used for superimposed images, curve fitting at turning points, noise removal or image texture modification. The control circuit 50 is coupled to the display unit 70 through a transmission line 80, so that the stitched image 106 is output to the display unit 70 through the transmission line 80.

The triple-axis gyroscope 60 is fixed in a housing 2 coupled to the second plane 12, and outputs a three-dimensional signal 107 to the control circuit 50. The control circuit 50 receives the plurality of the image signal 103, the plurality of the displacement signal 104, the skin mark 105, and the three-dimensional signal 107. The control circuit 50 stitches the plurality of the image signals 103 into the stitched image 106 with an actual curvature according to the plurality of the displacement signals 104, the skin mark 105, and a plurality of the three-dimensional signals 107. The actual curvature may be a true curvature of a real human face surface of the current user, and the actual curvature may be obtained by actually measuring a plurality of three-dimensional signals 107 on the human face surface of the current user. In a first embodiment of the present disclosure, the three-dimensional signal 107 includes a three-dimensional value of (a, b, c), and each of the values of a, b, and c represents a rotation angle in each direction relative to the preset absolute zero point (as shown in FIG. 2). Further, when the control circuit 50 receives one of the pluralities of the image signals 103, the control circuit (50) determines whether another one of the pluralities of the image signals 103 is continuously received according to a threshold value and at least one of the pluralities of the displacement signals 104. In the first embodiment of the present disclosure, the threshold value may be 2mm. That is, when the control circuit 50 determines that the image signal 103 has been displaced by 2mm from current position, the control circuit 50 receives another image signal 103 for image stitching. Because the definition of the coordinates is based on one FOV as a point (for example, the center point of 3mm x 3mm), two consecutive image signals 103 are overlapped with a distance of 2mm to generate the aforementioned 1mm overlap area.

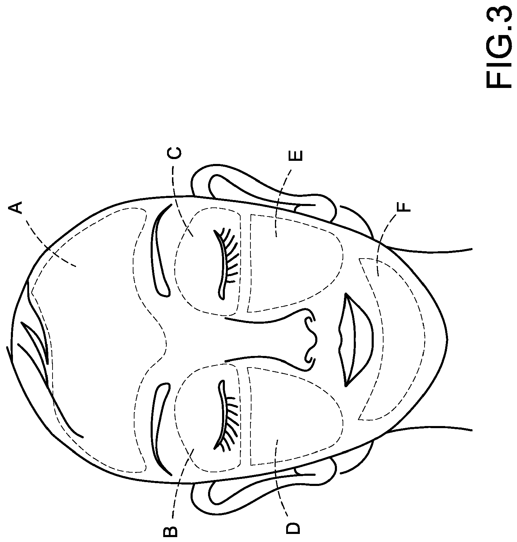

Please refer to FIG. 3, which is a schematic diagram showing skin marks of the microscopic imaging stitching apparatus of the present disclosure. Each skin mark 105 includes a preset curvature of each part of the skin to be tested 1. In the first embodiment of the present disclosure, the skin mark 105 may include a forehead A, a right eye B, a left eye C, a right cheek D, a left cheek E, and a chin F. It is considered that in actual detection of human skin, it is not necessary to perform a complete inspection of the entire face or even the whole body skin to be able to show that the current detection area is located at the correct relative position of the human body. Therefore, the skin mark 105 can be input before or simultaneously with the detection of the skin to be tested 1. Because the control circuit 50 can know where the part of human body currently being detected through the skin mark 105, even if not completely detected the entire face or whole body skin, the display unit 70 can still display the stitched image 106 of part of human skin on a virtual facial image according to the difference in the preset curvature of each skin mark 105 for each skin to be tested 1. For example, when the skin mark 105 is the right eye B, the stitched image 106 presented by the display unit 70 will not appear as the left eye C due to misjudgment. Furthermore, the present disclosure can combine the preset curvature corresponding to each skin to be tested 1 and the three-dimensional signal 107 output by the triple-axis gyroscope 60. Therefore, the microscopic imaging stitching apparatus of the present disclosure can mutually match the virtual face image and the three-dimensional values a, b, and c obtained from the real detected skin to be tested 1, and partially combined with each other to form a face image more precise. Alternatively, based on the virtual face image, the virtual face image may be modified according to the three-dimensional values a, b, and c obtained from the real detected the skin to be tested 1, thereby presenting the stitched image 106 that conforms to the real situation. That is, the control circuit 50 can synchronously correct the preset curvature in real time according to the actual curvature. However, the foregoing is merely illustrative, and there is not limited an application of the present disclosure.

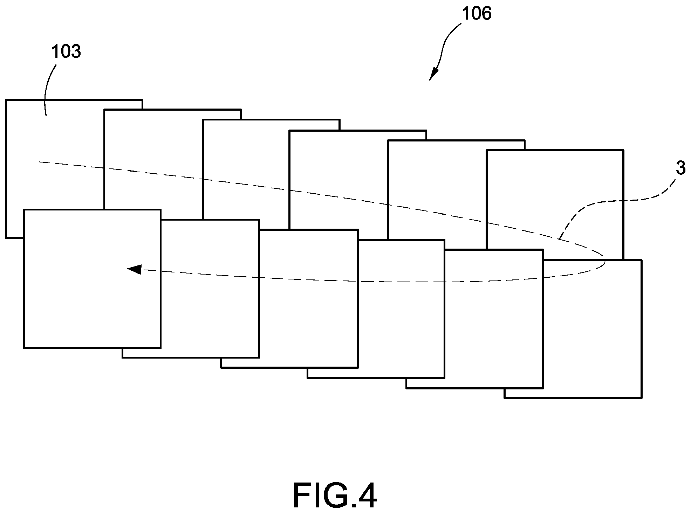

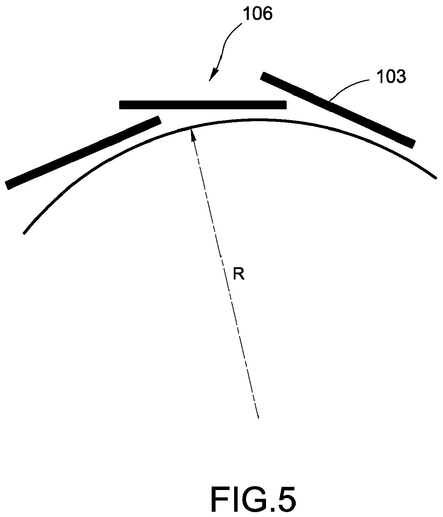

Please refer to FIG. 4 and FIG. 5, which are schematic diagrams of the stitching images of the microscopic imaging stitching device of the present disclosure. When using the microscopic imaging stitching apparatus of the present disclosure, the control circuit 50 can perform image stitching under a condition that the image signal 103 must be continuous. As shown in FIG. 4, in a moving path 3 of the first plane 11 on the skin to be tested 1, the plurality of the image signals 103 are sequentially stitched and partially overlapped. When encountering the non-planar of the skin to be tested 1, the control circuit 50 corrects the preset curvature (i.e., 1/R, where R is a radius of curvature) of the plurality of the image signals 103 in real time according to the skin mark 105 and the three-dimensional signal 107. The control circuit 50 may perform curve fitting on a plurality of the image signals 103 at a turning point, so as to generate smooth curves, or may achieve an effect of signal noise reduction.

The triple-axis gyroscope 60 is used to measure an absolute rotation angle of each FOV (for example, 3mm x 3mm) or a relative rotation angle from the previous image signal 103, and more considering the X and Y displacements. The five physical quantities (X, Y, a, b, c) can be integrated to stitch a three-dimensional surface image. It's a bit like a three-dimensional puzzle. Each part of the three-dimensional puzzle has its own FOV taken alone by itself. Because each scan is an independent event, that is, the origin (absolute zero) of re-zeroing, when the image sensor 30 takes the first picture and obtains an origin point (x=0, y=0, a=0, b=0, c=0), it starts to move. If it is defined by software or algorithm to take another photo every 2mm move of the FOV, and the displacement sensor 40 has moved from the absolute zero to more than 2mm, the control circuit 50 is triggered to capture another image signal 103, the image sensor 30 is caused to perform a photographing operation. At the same time, the control circuit 50 also captures the three-dimensional signal 107 of the triple-axis gyroscope 60. That is, information such as (x=2, y=1, a=1, b=1, c=1) is obtained, so that the control circuit 50 stitches two consecutive image signals 103 in three-dimensional space.

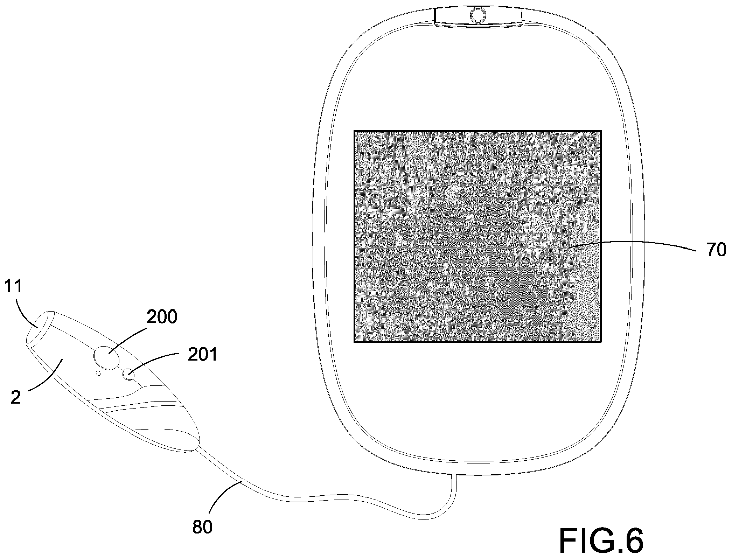

Please refer to FIG. 6, which is a schematic diagram of a second embodiment of the microscopic imaging stitching apparatus of the present disclosure. The second embodiment of the present disclosure is substantially the same as the first embodiment aforementioned, except that the housing 2 is covered outside the annular surface 13 to expose the first plane 11, and a button 200 and an indicator lamp 201 are arranged outside the housing 2. The button 200 allows user to define a starting point and an ending point for scanning and detecting the skin to be tested 1. The indicator lamp 201 can display current detection mode or power status. The button 200 can be a physical button, and the button 200 can also be replaced by a virtual button on the software. However, the foregoing is merely illustrative, and there is not limited an application of the present disclosure.

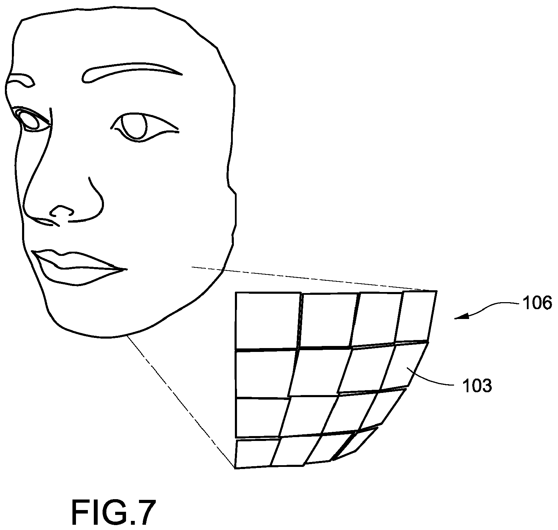

When using the microscopic imaging stitching apparatus of the present disclosure, the optical cavity 10 receives the reflected light 100 reflected from the skin to be tested 1 through the microscope lens 121. And then the beam splitter 20 outputs the first light to be tested 101 to an image sensor 30 (such as a CCD or CMOS), and outputs the second light to be tested 102 to the displacement sensor 40 (such as an optical XY two-dimensional sensor). Because of most of energy of the reflected light 100 is distributed to the image sensor 30, it will not affect an establishment of subsequent stitching images 106. And because of the displacement sensor 40 and the image sensor 30 can be synchronously performed (if the control circuit 50 determines that the displacement signal 104 is larger than the threshold value), the control circuit 50 can evenly and smoothly perform stitching processing on the plurality of image signals 103. In detail, the skin mark 105 corresponding to each part of the skin to be tested 1 can be inputted or preset to store before measurement. Because each skin mark 105 includes the curvature of each part of the skin to be tested 1 (for example, a curvature of chin skin of human is larger than a curvature of forehead skin of human). The control circuit 50 can stitch the plurality of image signals 103 into the stitched image 106 with a curvature according to the plurality of displacement signals 104 and the at least one skin mark 105. The curvature can be a preset value that conforms to most human skin types, and can enable the control circuit 50 to obtain the stitched image 106 with less perspective distortion than a traditional fully flat image. It effectively reduces the misjudgment of skin geometric features. When encountering the non-planar of the skin to be tested 1, the control circuit 50 corrects the preset curvature (1/R) of the plurality of the image signals 103 in real time according to the skin mark 105 and the three-dimensional signal 107. Please refer to FIG. 7, which is a schematic diagram of facial skin imaging of the microscopic imaging stitching apparatus of the present disclosure.

For the interpretation and analysis of microscopic images of dermatology, the present disclosure can provide an unprecedented way of observation, which overcomes shortcomings of traditional digital which only sees a small area but does not see a large area in microscopic images. At the same time, it is technically abandoning purely image features to do image stitching. The present disclosure can know the spatial distribution of the actual image with the actual physical quantity parameters (X, Y, a, b, c), and then construct a three-dimensional surface distribution model of real size. It can facilitate visual interpretation, comparison, and tracking, and provide more image information.

For this reason, the microscopic imaging stitching apparatus that solves the technical problems in prior art, so it can present a single microscopic image with several times FOV of single digital microscopic apparatus and can overcome the perspective distortion of skin's geometric features. Thereby achieving the purpose of facilitate the analysis of large-scale microscopic images and improving an accuracy of analysis.

## Claims

1. A microscopic imaging stitching apparatus used for detecting a skin to be tested (1), **characterized in that** the microscopic imaging stitching apparatus comprising:
an optical cavity (10) having a frustum of a cone with a first plane (11) and a second plane (12) arranged parallel to each other, an area of the first plane (11) smaller than an area of the second plane (12), and the area of the first plane (11) contacted the skin to be tested (1), the second plane (12) configured to emit light to the first plane (11) and configured to receive a reflected light (100) reflected from the skin to be tested (1),
a beam splitter (20) configured to receive the reflected light (100) through a microscope lens (121), and configured to output a first light to be tested (101) and a second light to be tested (102) along two different directions,
an image sensor (30) configured to receive the first light to be tested (101), and configured to output an image signal (103),
a displacement sensor (40) configured to receive the second light to be tested (102), and configured to output a displacement signal (104), and
a control circuit (50) coupled to the image sensor (30) and the displacement sensor (40), the control circuit (50) configured to receive the image signal (103) and the displacement signal (104), and configured to stitch a plurality of the image signals (103) into a stitched image (106) according to a plurality of the displacement signals (104).

2. The microscopic imaging stitching apparatus in claim 1, wherein the control circuit (50) is configured to further receive at least one skin mark (105), each of the at least one skin mark (105) includes a preset curvature corresponding to each skin to be tested (1), and the control circuit (50) is configured to stitch the plurality of the image signals (103) into the stitched image (106) with the preset curvature according to the plurality of the displacement signals (104) and the at least one skin mark (105).

3. The microscopic imaging stitching apparatus in claim 1, further comprising a triple-axis gyroscope (60) coupled to the control circuit (50), the triple-axis gyroscope (60) fixed in a housing (2) and configured to output a three-dimensional signal (107) to the control circuit (50), the control circuit (50) configured to receive the image signal (103), the displacement signal (104), and the three-dimensional signal (107), and the control circuit (50) configured to stitch the plurality of the image signals (103) into the stitched image (106) with an actual curvature according to the plurality of the displacement signals (104) and a plurality of the three-dimensional signals (107).

4. The microscopic imaging stitching apparatus in claim 1, further comprising a plurality of light-emitting units (122) arranged on the second plane (12), the plurality of the light-emitting units (122) configured to emit light toward the first plane (11), a minimum angle between an extension line of one side of each of the plurality of the light-emitting units (122) and the normal line of the first plane (11) being 30°.

5. The microscopic imaging stitching apparatus in claim 1, wherein when the control circuit (50) is configured to receive one of the plurality of the image signals (103), the control circuit (50) is configured to determine whether another one of the plurality of the image signals (103) is continuously received according to a threshold value and at least one of the plurality of the displacement signals (104).

6. The microscopic imaging stitching apparatus in claim 5, wherein the threshold value is 2mm, and the field of view (FOV) of the image sensor (30) is 3mm x 3mm.

7. The microscopic imaging stitching apparatus in claim 1, wherein a ratio of a light intensity of the first light to be tested (101) to a light intensity of the reflected light (100) is 99%, 95%, or 90%, a ratio of a light intensity of the second light to be tested (102) to a light intensity of the reflected light (100) is 1%, 5%, or 10%.

8. A microscopic imaging stitching method for detecting a skin to be tested (1), **characterized in that** the microscopic imaging stitching method comprising following steps of:
emitting, by a frustum of a cone, light to a skin to be tested (1),
receiving, by a beam splitter (20), a reflected light (100) reflected from the skin to be tested (1) through a microscope lens (121), and outputting a first light to be tested (101) and a second light to be tested (102) along two different directions,
receiving, by an image sensor (30), the first light to be tested (101), and outputting an image signal (103),
receiving, by a displacement sensor (40), the second light to be tested (102), and outputting a displacement signal (104), and
receiving, by a control circuit (50), the image signal (103) and the displacement signal (104), and stitching a plurality of the image signals (103) into a stitched image (106) according to a plurality of the displacement signals (104).

9. The microscopic imaging stitching method in claim 8, wherein the control circuit (50) is configured to further receive at least one skin mark (105), each of the at least one skin mark (105) includes a preset curvature corresponding to each skin to be tested (1), and the control circuit (50) stitches the plurality of the image signals (103) into the stitched image (106) with the preset curvature according to the plurality of the displacement signals (104) and the at least one skin mark (105).

10. The microscopic imaging stitching method in claim 8, wherein the control circuit (50) further received a three-dimensional signal (107), and the control circuit (50) stitches the plurality of the image signals (103) into the stitched image (106) with an actual curvature according to the plurality of the displacement signals (104) and a plurality of the three-dimensional signals (107).

11. The microscopic imaging stitching method in claim 8, further comprising following step of:
determining, the control circuit (50), whether another one of the plurality of the image signals (103) is continuously received according to a threshold value and at least one of the plurality of the displacement signals (104) when the control circuit (50) configured to receive one of the plurality of the image signals (103).

12. The microscopic imaging stitching method in claim 11, wherein the threshold value is 2mm, and the field of view (FOV) of the image sensor (30) is 3mm x 3mm.

13. The microscopic imaging stitching method in claim 11, wherein an area of 1mm inward around the FOV of the image sensor (30) is an overlap area that one of the pluralities of the image signals (103) and another image signal (103) are stitched together.

14. The microscopic imaging stitching method in claim 8, wherein a ratio of a light intensity of the first light to be tested (101) to a light intensity of the reflected light (100) is 99%, 95%, or 90%, a ratio of a light intensity of the second light to be tested (102) to a light intensity of the reflected light (100) is 1%, 5%, or 10%.

15. The microscopic imaging stitching method in claim 8, further comprising following step of:
moving, by the frustum of a cone, on the skin to be tested (1) at a speed of 1mm/s.
